# EUROPEAN PATENT SPECIFICATION

(11) **EP 2 889 604 B1**
(45) Date of publication and mention of the grant of the patent: **15.12.2021**
(21) Application number: 14200441.5
(22) Date of filing: 29.12.2014
(51) Int. Cl.: G01N 33/46, G01N 21/3563, G01N 21/359, G01N 21/85, G01N 21/94

(54) **Method to evaluate the quality of cork and corresponding evaluation apparatus**
Verfahren zur Beurteilung der Qualität von Kork und entsprechende Vorrichtung
Procédé d'évaluer la qualité de liège et dispositif correspondant

(30) Priority: 30.12.2013 IT UD20130175
(43) Date of publication of application: 01.07.2015
(73) Proprietor: Universita' Degli Studi di Udine, 33100 Udine (IT)
(72) Inventor: Battistutta, Franco, 33050 Rivignano (UD) (IT); Brotto, Laura, 34070 San Pier d'Isonzo (GO) (IT); Zironi, Roberto, 33100 Udine (IT)
(74) Representative: Petraz, Gilberto Luigi

(56) References cited:
- EP-A1- 2 518 478
- US-A1- 2005 092 112
- YEW LI HOR ET AL: "Nondestructive evaluation of cork enclosures using terahertz/millimeter wave spectroscopy and imaging", APPLIED OPTICS, OPTICAL SOCIETY OF AMERICA, WASHINGTON, DC; US, vol. 47, no. 1, 1 January 2008 (2008-01-01), pages 72-78, XP001510998, ISSN: 0003-6935, DOI: 10.1364/AO.47.000072
- CRISTINA PRADES ET AL: "Methodology for cork plank characterization (Quercus suber L.) by near-infrared spectroscopy and image analysis", MEASUREMENT SCIENCE AND TECHNOLOGY, IOP, BRISTOL, GB, vol. 21, no. 6, 28 April 2010 (2010-04-28) , page 65602, XP020192834, ISSN: 0957-0233, DOI: 10.1088/0957-0233/21/6/065602
- ANA R. GARCIA ET AL: "The Problem of 2,4,6-Trichloroanisole in Cork Planks Studied by Attenuated Total Reflection Infrared Spectroscopy: Proof of Concept", JOURNAL OF AGRICULTURAL AND FOOD CHEMISTRY, vol. 63, no. 1, 9 December 2014 (2014-12-09), pages 128-135, XP055174284, ISSN: 0021-8561, DOI: 10.1021/jf503309a
- ISABEL MARTINS ET AL: "Investigating Aspergillus nidulans secretome during colonisation of cork cell walls", JOURNAL OF PROTEOMICS, vol. 98, 4 December 2013 (2013-12-04), pages 175-188, XP055113083, ISSN: 1874-3919, DOI: 10.1016/j.jprot.2013.11.023

## Description

### FIELD OF THE INVENTION

The present invention concerns a method to evaluate the quality of cork intended mainly, but not exclusively, for use in the food sector, for example in the wine-making sector.

A possible application of the present invention is the evaluation of the quality of the cork intended for use as corks or stoppers.

The present invention also concerns an apparatus to evaluate the quality of the cork.

With the method and the apparatus according to the present invention it is possible to select the cork substantially automatically, depending on the quality detected, in order to divide it into pre-established quality categories.

### BACKGROUND OF THE INVENTION

It is known that it is possible to obtain wood materials from the trunk of a tree that is subsequently intended for use in different areas.

One of the possible applications is in the food sector for example: to this purpose, to preserve the organoleptic properties of food products that can interact with the wood material, different methods have been adopted to evaluate the quality of the wood material so as to evaluate its suitability for use.

One of the possible applications of the wood material concerns cork, which is used to make stoppers to close bottles or containers in general.

The use of stoppers, made of natural cork and synthetic cork, to close wine bottles is not without risk and can cause conditioning phenomena of an organoleptic nature linked to the transfer of undesired compounds (anisoles, in particular 2,4,6-trichloroanisole or TCA), which causes anomalous odors to develop, known as "cork taint". The appearance of these alterations causes huge economic losses and considerable damage to the image of both wine producers and cork mills, which are frequently involved in legal disputes which go on for years. 2,4,6- trichloroanisole derives from the fungal metabolism and is therefore associated to the development of molds on the cork (directly on the plant or during the production process).

Given the very low concentrations at which 2,4,6- trichloroanisole causes the onset of the defect (1-4 ng/L in wine), the analytical methods to determine it are complex, very costly and often ineffective. Moreover, the uneven nature of pollution from 2,4,6- trichloroanisole (localized in the areas of the planks in which there has been a fungal attack) renders it difficult to provide sampling plans that allow to obtain a reliable evaluation of the quality of the individual batches.

The formation path of 2,4,6- trichloroanisole in cork is rather complex and is fundamentally linked to the presence of substances with a chlorine base (phytosanitary products, environmental pollution) and to the development of fungal species that are able to methylate the chlorophenols to chloroanisoles. At an industrial level, to prevent the development of molds and reduce the effect of the defect, a whole series of precautions have been perfected that have led to numerous innovations in the production line of cork (abolition of chlorine-based phytosanitary products in the cork forests, abandoning hypochlorite washing systems) and a series of systems for cleaning the cork have been perfected, using steam (ROSA method), super-critical CO₂, microwaves (DELFIN^{®} method), able to abate the concentration of 2,4,6- trichloroanisole. However, these methods are only effective on granular or ground cork.

Despite the numerous ways followed to contain the problem, currently the problem of pollution from 2,4,6- trichloroanisole involves percentages comprised between 2% and 5% of the production of cork stoppers. At present, no methods of analysis are known that allow to evaluate quickly, economically and non-destructively the content of 2,4,6- trichloroanisole in cork stoppers.

The most widespread analytical methods to evaluate this type of contaminants are based on solid-liquid extraction, concentration of the extract and injection into a system of mass gas-chromatography-spectrometry or on using solid-phase micro extraction and subsequent injection into mass gas-chromatography-spectrometry systems or into an electron capture detector.

In the industrial field, over the last thirty years, a whole series of analytical techniques have been developed, based on spectroscopy in the near and mid infrared, which allow to study quickly and economically the quality characteristics of the products. NIR, FT-NIR and MIR spectroscopies can be comprised as spectroscopy in the infrared band.

With spectroscopy in the near-infrared it is possible to record the response of the molecular bonds of the chemical constituents subjected to radiations comprised in a range from 4000 to 12000 cm⁻¹ (for example of the O-H, N-H C-H bonds) and in this way obtain a characteristic spectrum that acts as a "digital fingerprint" of the sample analyzed.

This technique has numerous advantages because, as it is a non-destructive technique, it offers the possibility of analyzing the sample as such, it allows to perform quickly both quality and quantity analyses on samples in any physical state whatsoever (solid, crystalline, micro-crystalline, amorphous, liquid or gas) and, thanks to the use of fiber-optics or suitably prepared systems, it is suitable for using instruments for in-line analysis. Using spectroscopy in the near-infrared, for quality-quantity analyses in the industrial sector, is closely connected to the development and use of multivariate statistical data processing techniques. Possible statistical data processing techniques can comprise for example *Principal Component Analysis,* or PCA, or *Partial Least Squares* or PLS, or SIMCA.

NIR spectrums contain a large quantity of information relating to the composition and structure of the sample, but this information cannot be correlated simply and directly with an analytical datum unless complex statistical data processing systems are used, which allow to separate the information from the noise and which take into account the interaction between the variables. In the food sector, spectroscopy in the near-infrared has been widely used for years for the in-line analysis of the composition in macro constituents (humidity, fats, proteins etc.) of numerous products (cereals, milk derivatives, meat, etc.).

A method is also known, from EP2518478 A1, to detect intrinsic parameters of cork stoppers that is based on non-invasive, non-destructive techniques. In particular, the known method is based on X-ray spectroscopy techniques. Depending on the spectroscopic analysis, the stoppers can be categorized according to parameters that are considered relevant for the closing capacity, for example gas permeability.

In this case, using X-ray spectroscopy techniques is suitable for detecting physical properties of the product, which is, precisely, the gas permeability, but it is totally ineffective for evaluating organoleptic properties. The alterations of physical properties, in fact, are macroscopic entities that can be detected directly using X-ray spectroscopy techniques, whereas organoleptic properties, also because of the limited presence of components that determine said properties, are not directly detectable with spectroscopy techniques and in particular cannot be detected with X-ray spectroscopy techniques.

From the article by Yew Li Hor et al "Nondestructive evaluation of cork enclosures using terahertz/millimeter wave spectroscopy and imaging", Applied Optics, Optical Society of America, Washington, DC; US, vol. 47, no.1, (2008-01-01), pp. 72-78, a non-destructive method is known for evaluating structural properties of cork such as cracks, defects, grain modifications. The spectroscopic analyses described in this document suggest adopting wavelengths in the order of 1mm. The article of Yew Li Hor discloses moreover the detection of 2,4,6-TCA in cork, which is linked to a fungal attack. The detection is based on THz time domain spectroscopy.

The characteristic spectrums detectable with this method are not able to supply sufficient information to determine the organoleptic properties of the cork.

Another article by Cristina Prades et al "Methodology for cork plank characterization (Quercus suber L.) by near-infrared spectroscopy and image analysis; Methodology for cork plank characterization (Quercus suber L.) by NIRS and image analysis", Measurement Science and Technology, IOP, Bristol, GB, vol. 21, no.6 (2010-04-28), p. 65602, describes a method based on spectroscopic analysis in the near-infrared to evaluate the physical properties of cork such as visual quality, porosity, humidity and the geographical origin of the cork. This article does not allow to identify if the cork is contaminated by a fungal attack.

In fact, spectroscopic analysis in this case is intended only to evaluate/detect data inherent to the physical properties of the cork, and therefore macroscopic entities, not microscopic entities like those connected to contamination caused by fungal attack.

The method of spectroscopic analysis in the near-infrared described in this article cannot be transferred directly to an evaluation of a fungal attack, since the quantitative analysis of the micro-constituents (aromatic fraction, contaminants...) is particularly complex due to the low concentrations at which these compounds are present in foodstuffs, and due to the fact that the micro-constituents do not significantly modify the response of the molecular bonds to infrared radiation, and therefore the physical properties of the cork.

In the cereals sector a series of analytical techniques have been perfected, to evaluate the contamination from myco-toxins, pollutants deriving from the fungal metabolism, present in extremely low concentrations in foods attacked by molds, which are very dangerous for the health of humans and animals and are subject to extremely strict legal limits. The evaluations proposed are semi-quantitative, since the variations in the NIR spectrum used for constructing the regression models are due more to the alteration of the macro-compositive structure of the product for which the molds are responsible than the presence of myco-toxins, generally present in concentrations that are too low to be seen with this type of technique.

Moreover, Isabel Martins et al. "Investigating Aspergillus nidulans secretome during colonization of cork cell walls", Journal of Proteomics, vol. 98, 4 December 2013, pages 175-188, discloses Attenuated Total Reflectance-Fourier Transform Infrared Spectroscopy in the range of 4000-600 cm⁻¹ for detecting fungal cultures in cork samples.

One purpose of the present invention is to perfect a method to evaluate, and possibly also to select, the quality of cork that provides objective analysis information.

Another purpose of the present invention is to perfect a method to evaluate the quality of cork that is not invasive, that is, it does not entail the destruction of the product analyzed.

Another purpose of the present invention is to perfect a method to evaluate the quality of cork that allows to carry out accurate tests, not random statistics, on each material analyzed.

Another purpose of the present invention is to provide a method to evaluate the quality of cork that is quick and that supplies the results of the analysis carried out in real time.

Another purpose of the present invention is to produce an apparatus to evaluate the quality of cork that uses the evaluation method of the present invention.

Another purpose of the present invention is to produce an apparatus to evaluate the quality of cork that is simple and economical.

Another purpose of the present invention is to produce an apparatus to evaluate the quality of cork that allows to perform analyses substantially in-line.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe further characteristics of the invention .

The method according to the invention is defined by claim 1 and the apparatus according to the invention is defined by claim 10.

In this way the method and the apparatus to evaluate the quality of cork allow to identify an objective methodology to evaluate the quality of cork, in relation to parameters that condition the quality of the wood material.

Indeed, the method provides to use calibration samples analyzed using a known technique, for example sensory or chromatographic techniques or possibly, with the integration of both the analyses, to detect their characteristic spectrums and to associate these sample spectrums, both non-contaminated and contaminated with known contamination values, to respective quality categories.

By processing these spectrums of the calibration samples reference parameters are determined that allow to identify/characterize one category of corks with respect to another, for example a category of polluted or non-polluted products, or with high or low levels of pollution.

These reference parameters act as comparison parameters with conditioning parameters that are detected in the products made of cork that are to be evaluated.

In particular, the conditioning parameters are evaluated with processing modes similar to those carried out previously to evaluate the reference parameter.

The method and the apparatus allow to obtain a direct and in-line evaluation of all the cork products processed, and therefore allow to obtain an objective evaluation technique of the cork.

With the present invention it is therefore possible to supply a method and an apparatus able to evaluate whether the cork has been attacked by conditioning phenomena of an organoleptic nature, for example the onset of molds.

The present invention allows to identify if the product analyzed can interact with a food product without compromising its organoleptic properties. The processing of the data, possibly by means of multivariate statistical techniques allows to construct a predictive model to be used in the in-line evaluation and selection of the product.

The method and apparatus according to the present invention can be used on all products made of cork, not limited to a sample analysis of products. This considerably increases the quality of the products used.

The method and the apparatus to evaluate the quality of the cork can be used to evaluate the finished product in itself. For example, it can be provided to use these techniques on the planks of cork in pre- and post-boiling, on the bands of cork and on the individual stoppers being worked and on the cork granules.

With the present invention it is therefore possible to make available to producers of cork products, for example cork mills, a system to control the quality and to select the samples, depending on the presence of active organoleptic substances, applicable systematically to the whole production.

It is also possible to lower the costs of analyzing contaminants of the stoppers and to solve problems connected to the sampling of batches.

It is also possible to reduce the discards of raw material, thus containing production costs. Finally, it is possible to improve the overall quality of stoppers made of natural cork and to reduce the risk associated to defects that can be traced back for example to "cork taint" in wines, as well as giving objective evaluations of the characteristics of the wood.

A variant of the present invention provides that from the spectroscopic sampling analysis a plurality of reference parameters are identified that are characteristic of one quality category rather than another category, and that these reference parameters are memorized to constitute a database to be used as a basis for comparison for the spectroscopic analysis and the processing of the characteristic spectrum of the products made of cork.

### BRIEF DESCRIPTION OF THE DRAWINGS

These and other characteristics of the present invention will become apparent from the following description of some forms of embodiment, given as a non-restrictive example with reference to the attached drawings wherein:
- fig. 1 is a schematic representation of an apparatus to evaluate the quality of cork according to a possible form of embodiment of the invention;
- fig. 2 is a graphical representation of characteristic spectrums of products made of cork;
- fig. 3 is a graphical representation of results obtained with the evaluation method according to the present invention;
- fig. 4 is a graphical representation of results obtained with the evaluation method in accordance with possible embodiments of the invention;
- figs. 5 and 6 are graphical representations of two components of the PCA analysis.

To facilitate comprehension, the same reference numbers have been used, where possible, to identify identical common elements in the drawings. It is understood that elements and characteristics of one form of embodiment can conveniently be incorporated into other forms of embodiment without further clarifications.

### DETAILED DESCRIPTION OF SOME FORMS OF EMBODIMENT

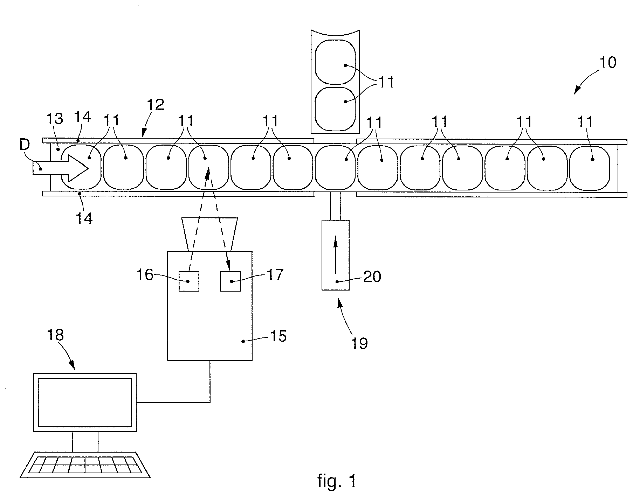

Fig. 1 is used to describe a possible form of embodiment of an apparatus 10 to evaluate the quality of the cork that makes up at least one part of at least one product 11 used preferably, but not exclusively, in the wine-making sector.

Possible if not exclusive applications of cork can be for making lids or stoppers for bottles or containers in general, used for example to contain food products, such as wine. The identification of quality can be made directly on the planks of cut bark, on the granules deriving from grinding the cork, on the finished product made of cork, such as a stopper, a lid or similar.

In accordance with a possible form of embodiment, the apparatus 10 according to the present invention comprises a support unit 12 on which to dispose the products 11, in this case stoppers made of cork, in order to evaluate their quality correlated to conditioning phenomena of an organoleptic nature.

The support unit 12 can possibly be configured to move the products 11 in a determinate direction of feed D.

In possible forms of embodiment the support unit 12 can comprise at least one of either a support plane 13, a conveyor belt, conveying walls 14, a thruster member, a movement device, or possible combinations thereof.

According to the invention, the apparatus 10 comprises a spectroscopic analysis device 15 configured to emit an optical ray, or electromagnetic beam, in the field of near-infrared toward at least a product 11 to be analyzed and to detect an optical ray or electromagnetic beam deriving from the interaction with the product 11 to be analyzed.

The optical ray deriving from the interaction with the product 11 to be analyzed in practice reproduces a characteristic spectrum of the zone of the product 11 that has been involved in the interaction with the optical ray.

In possible forms of embodiment, shown in fig. 1 for example, the optical ray deriving from the interaction with the product 11 to be analyzed is, for example, the optical ray that is reflected by the product 11.

According to the invention, the spectroscopic analysis device 15 is configured to carry out a spectroscopic analysis in the band of wavelengths in the near-infrared, also known as NIR. The bands of wavelengths in the near-infrared can be comprised between about 12800 cm⁻¹ and 4000 cm⁻¹, that is, comprised between about 780nm and about 2500nm.

The spectroscopic analysis device 15 comprises an emitter source 16 configured to emit an optical ray in the near-infrared field and a detection member 17, configured to receive the optical ray from the product 11 and detect the characteristic spectrum of the product analyzed.

In accordance with possible variants, the emitter source 16 can be based on the use of contact techniques, for example based on fiber optical probes, or based on distance techniques, for example comprising an illuminant in the infrared field and a remote sensor.

In accordance with a possible solution, the detection member 17 is configured to detect the optical ray reflected by the product 11.

In accordance with possible forms of embodiment, the spectroscopic analysis device 15 can be mounted laterally, above or below the support unit 12 in order to produce the optical ray toward the product 11 to be analyzed.

Some solutions of the invention provide that the spectroscopic analysis device 15 can be attached, that is supported by a frame, or manually movable.

In accordance with the invention, the apparatus 10 also comprises an electronic processing device 18 connected to the spectroscopic analysis device 15 and configured to acquire and process the characteristic spectrum of the product 11 to be analyzed.

The processing device 18 can comprise, for example, a calculator, a PLC, an electronic unit to collect, memorize, manage and process the data.

In possible solutions, the processing device 18 can also comprise a user interface unit through which a user can interact with the processing device 18 for the correct functioning of the apparatus 10.

The apparatus 10 is provided with at least one selector device 19 configured to select the products 11 which have been previously analyzed with the spectroscopic analysis device 15 in relation to the quality detected.

In possible solutions, the selector device 19 can comprise at least one of either an actuator 20, a pneumatic nozzle, removal pincers, a translating plate, a switch, or a possible combination thereof.

The selector device 19 receives activation commands from the processing device 18 also in relation to the outcomes of the analysis. The transmission of the activation commands can occur with or without wires, in wireless mode for example.

In the form of embodiment shown in fig. 1, the selector device 19 is mounted in-line along the support unit 12 and downstream of the spectroscopic analysis device 15.

If the processing device 18 determines a predefined quality of the product 11 in transit, for example that it is not suitable for use in the food sector, it commands the activation of the selector device 19 to transfer it outside the analysis line.

The presence of one or more selector devices 19 allows to categorize the different products 11 into different quality categories and to divide them suitably in relation to their level of quality.

The present invention also concerns the method to evaluate the quality of the cork that can possibly be implemented on an apparatus 10 as described above.

A method according to claim 1 is used to evaluate the quality of the cork correlated to conditioning phenomena of an organoleptic nature .

The spectroscopic analysis provides at least the emission of an optical ray toward the product 11 to be analyzed and the detection of the characteristic spectrum deriving from the interaction of the optical ray with the product 11 to be analyzed.

According to the invention, the optical ray is comprised in the band of wavelengths in the near-infrared, also known as NIR. To this purpose the corresponding detection sensor is also configured to acquire the ray in the field of near-infrared. These types of wavelength are particularly suitable to evaluate the quality of cork.

From the emission of the optical ray it is possible to detect the characteristic spectrum of the material that makes up at least part of the product 11 in order to subsequently identify the quality thereof.

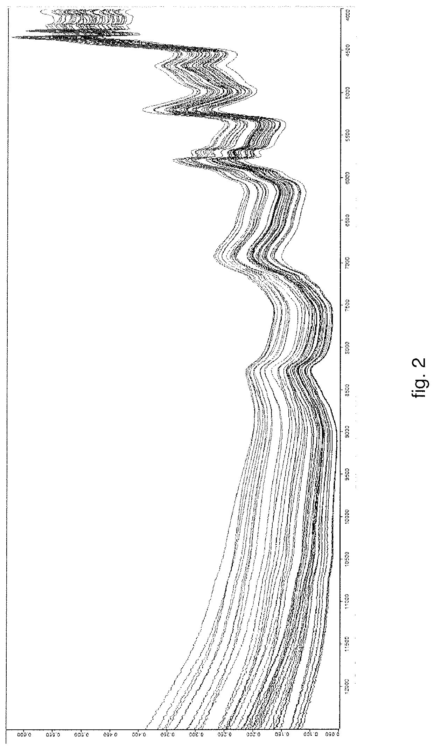

Possible characteristic spectrums of products 11 are shown by way of example in fig. 2.

The characteristic spectrums detected are processed, by the processing device 18, to evaluate the conditioning parameter or parameters of the quality of the cork.

In accordance with possible embodiments of the invention, the evaluation of the conditioning parameters of the quality of the cork provides to identify indexes of micro-structural/physical modification of the cork that condition its quality.

Within the framework of the analysis of the quality of the cork, the indexes of micro-structural/physical modification concern the fungal metabolism that has attacked the cork, causing pollution by anisoles and by other organoleptically active substances.

In accordance with other possible variants, the indexes of microstructural/physical modification concern the development of at least one of either anisoles, geosmin, isoborneol, guaiacol, octenone.

In possible embodiments of the present invention the indexes of micro-structural/physical modification concern the development of 2,4,6-trichloroanisole in cork.

In accordance with possible forms of embodiment of the invention, the processing of the characteristic spectrum of the cork provides to implement statistical processing techniques of the data detected in multivariate mode, to determine said conditioning parameters of the quality of the cork.

The method comprises an initial calibration step which, starting from calibration samples previously selected and divided into different quality categories, among which at least one that is indicative of contamination and one indicative of non-contamination, supplies reference parameters to be used as a comparison with said conditioning parameter.

The method according to the present invention comprises:
- supplying a plurality of calibration samples made of cork both contaminated and not contaminated by a fungal metabolism;
- dividing the calibration samples into a plurality of quality categories as a function of the attack by the fungal metabolism;
- spectroscopic sampling analysis in the near-infrared of the calibration samples in order to identify, by processing the respective characteristic spectrum, identifying reference parameters of each of the quality categories and correlated to the attack by the fungal metabolism.

Some possible implementations provide that the statistical processing techniques comprise at least one of either PCA "Principal Component Analysis", PLS "Partial Least Squares" or SIMCA.

The PCA technique is a statistical data processing method intended to extract the maximum information possible contained in a structure of multivariate data, synthesizing it in a few linear combinations of the variables.

This method is used in the first data processing step and serves to give a general view of the problem, to understand the relations between the objects and/or the classes considered and to supply a preliminary indication of the role of the variables. From the geometrical point of view, PCA consists of a rotation process of the original data, carried out so that the first new axis is orientated in the direction of the maximum variance of the data, the second is perpendicular to the first and is in the direction of the subsequent maximum variance of the data, and so on. The number of these new axes (principal components or factors) will be equal to the number of original variables. The first principal component will be able to explain the greatest percentage of variance, the second will explain a little less, the third even less and so on, until the last components contribute to explain little or nothing of the variability present in the data examined.

One feature of great importance in a study with multivariate analysis concerns the possibility of seeing the data graphically. In fact, PCA allows to graphically represent both the objects alone (cases or samples), by means of what is defined as *"score plot",* and also the variables alone by means of what is defined as *"loading plot",* and also both objects and variables simultaneously by means of what is defined as the "*biplot*", as a function of the values assumed by two of the principal components constructed by the mathematical function. For each principal component used in the construction of the graph, the percentage quantity of variance explained by the individual component is also expressed.

The PLS technique is a simple linear regression model that allows to study the linear relation between a response variable Y (called dependent or predictor variable) and an explicative variable (called independent or regressor variable). While the multiple linear regression is a variant of the previous one in which the response variable Y is studied starting from several explicative variables.

PLS is a multiple linear regression system in which, before constructing the straight line of regression, a factorization of the data is carried out (representation of the original data in a more efficient system of coordinates) by analyzing the principal components. The regression is not therefore constructed starting from the original data, but starting from factors obtained through an analysis of the principal components (PCA) carried out by taking into account both the values assumed by the explicative variables and also the values of the response variable. The first factor generally explains the greater variations; with the subsequent factors the residual variance is characterized.

The development of a multivariate method using the PLS technique provides to follow a precise sequence of operations:
1. accurate measuring of the spectrums of the calibration samples and the reference values relating to the variable/ reference parameter to be predicted;
2. verification of the set of data to identify the possible presence of outliers or incorrect samples. Possible outliers can be identified in different steps of the construction process of the model. In the initial step a PCA is carried out which allows to graphically identify samples that have a very different behavior from that of the rest of the dataset;
3. selection of the range of data on which to work (spectral range in the case of spectroscopic analyses) and choice of the type of pre-treatment to be applied to the data. The statistical softwares developed for the construction of PLS models starting from the spectral data offer a vast range of possibilities for pre-treating the data (Minimum-Maximum Normalization, Offset Correction, Vectorial Normalization, Multiplicative Scattering Correction, First Derivative, Second Derivative, also No Pre-treatment), that allow to amplify the differences between the samples;
   Generally the softwares dedicated to this type of analysis offer the possibility of automatically identifying the spectral zones and the type of pre-treatment of the data that allow to obtain the best models; the option is defined as optimization.
4. construction of the calibration model;
5. validation of the model; during this step of the process the capacity of the regression method identified to predict the value of the dependent variable in a set of unknown samples is evaluated. For validation it is possible to use the cross validation technique or the test set validation technique.

In the first case, validation is carried out with the same data-set of samples used for calibration, by excluding from the data-set one sample at a time and analyzing the rest of the samples using the model previously constructed. In the second case, the data-set is divided into two homogenous groups: one of the two groups is used for calibration in the construction of the model, whereas the other is used to evaluate the predictive effectiveness of the model. In this phase a series of parameters are calculated that are useful for evaluating the correctness and robustness of the regression model which has been constructed.

Multivariate statistical processing techniques allow to identify in the characteristic spectrum trends of the curves that are indexes of the quality of the cork.

According to the invention, the processing of the characteristic spectrums also provides to compare the conditioning parameters of the quality of the cork with the reference parameters.

According to possible forms of embodiment, the reference parameters can be determined as a function of tests made on samples of products with pre-coded qualities. According to possible forms of embodiment, the samples can be pre-coded using traditional/known techniques, for example sensory, of samples belonging to one quality category rather than another.

According to a variant, the sensory analysis can be integrated with and/or replaced by chromatographic techniques.

To this purpose, in order to evaluate the objectivity of the data detected, Applicant carried out a test on a reduced scale, using a laboratory FT-NIR spectrometer and applying data processing systems that allowed to construct a statistical model able to distinguish samples of cork polluted by 2,4,6-trichloroanisole from non-polluted samples of cork, based on the characteristics of the spectrum in the near-infrared. The tests were made using a series of samples of natural cork in the form of strips, divided into three quality categories as a function of pollution by 2,4,6-trichloroanisole. In particular, Applicant deemed it necessary to divide the samples at least into the quality categories of non-polluted, not very polluted, very polluted. From the analysis of the individual samples using an FT-NIR spectrometer, equipped with a reading system for solids that uses an integration sphere, a quality model was constructed that allows to distinguish the three groups of samples and to locate the unknown samples correctly in the corresponding group, depending on the TCA pollution.

In the framework of the analysis, multivariate statistical processing techniques were used, which allowed to identify graphically zones of samples belonging to one group rather than another.

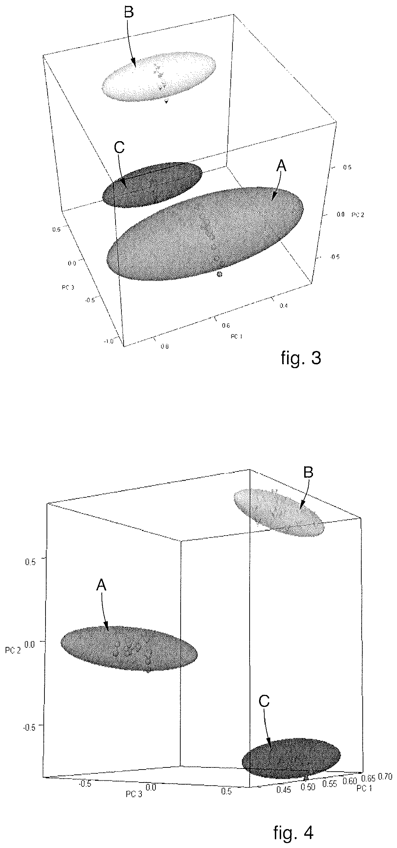

Fig. 3 shows a graph in which said three zones are identified, which respectively identify the non-polluted group (indicated by arrow A), the not very polluted group (indicated by arrow B), and the very polluted group (indicated by arrow C). According to possible solutions, the three zones or the mathematical geometric places that identify the three zones, can define the reference parameters to be used as comparison parameters for the conditioning parameters detected on the products 11.

According to one possible embodiment of the invention, the processing of the characteristic spectrum provides to extrapolate from it the data relating to a range of wavelengths.

According to one possible solution, the range of wavelengths of the characteristic spectrum considered is comprised between 4500 cm⁻¹ and 10000 cm⁻¹, although choosing a different range of wavelengths in the near-infrared band is not excluded.

In this range of wavelengths it is possible to make a first separation of the three quality classes of the cork (clean, not very polluted, extremely polluted) with a strong intervention on the first processing of the spectrum.

Inside the range it is possible to identify some zones of the spectrum that give much stronger indications.

During processing, the spectrums of both the calibration samples and the products 11 can be subjected to mathematical filters. The mathematical filters can comprise, by way of non-restrictive example of the present invention, at least one of either a second derivative or a vectorial normalization.

By way of example, fig. 4 shows a PCA carried out on a range 7486.7-919.6 cm⁻¹ with pre-treatment of the spectrum with second derivative. The better distribution of the data grouped in a cloud is obvious.

The corresponding data in the range of wavelengths selected can be filtered to eliminate unwanted disturbance or noise components, which could distort the results of the analysis.

From the filtered data it is possible to construct a characteristic function of the spectrum detected. The characteristic function is processed mathematically to extract from it characteristic components that can be correlated to the conditioning parameters of the quality of the cork.

In one possible solution, the mathematical processing of the characteristic function provides to calculate its second derivative so as to extrapolate characteristic peaks that correspond to the conditioning parameters and to the reference parameters of the quality of the cork.

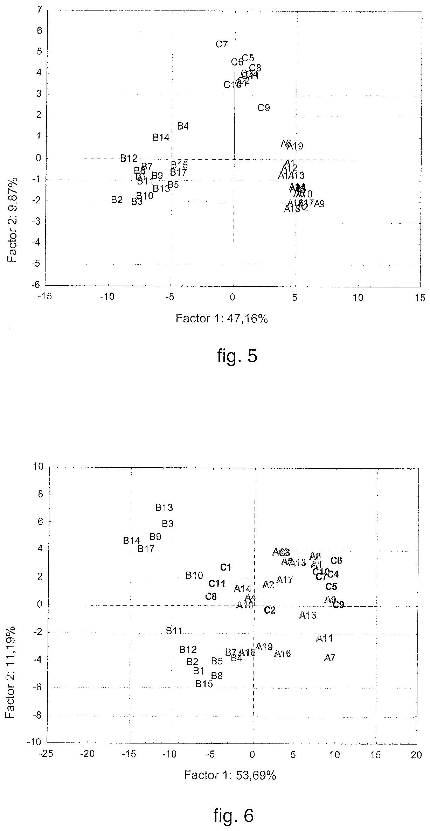

From the tests carried out, Applicant has extrapolated algorithms deriving from the PCA: the graphical results of the projection of the cases on the first two PCA components are shown in fig. 5, for a range of 11300-12450 cm⁻¹.

Hereafter, the weight of the variables on PCA factors for this analysis are shown.

| | Factor 1 | Factor 2 |
|---|---|---|
| 12439 | 0.015159 | 0.051541 |
| 12435 | 0.025144 | 0.027718 |
| 12412 | 0.010688 | 0.024270 |
| 12393 | 0.011600 | 0.009693 |
| 12374 | 0.017732 | 0.000932 |
| 12370 | 0.027103 | 0.001205 |
| 12366 | 0.028262 | 0.007897 |
| 12362 | 0.026476 | 0.016506 |
| 12351 | 0.012982 | 0.002785 |
| 12347 | 0.025665 | 0.000001 |
| 12343 | 0.024735 | 0.001458 |
| 12339 | 0.025425 | 0.015958 |
| 12327 | 0.012262 | 0.041143 |
| 12289 | 0.014496 | 0.042586 |
| 12285 | 0.015579 | 0.061800 |
| 12189 | 0.020212 | 0.000627 |
| 12185 | 0.018956 | 0.003488 |
| 12181 | 0.009942 | 0.001105 |
| 12169 | 0.020335 | 0.008442 |
| 12165 | 0.025072 | 0.000000 |
| 12162 | 0.019072 | 0.002050 |
| 12158 | 0.010430 | 0.000008 |
| 12142 | 0.016068 | 0.003932 |
| 12138 | 0.016602 | 0.000045 |
| 12042 | 0.010895 | 0.040344 |
| 12038 | 0.016274 | 0.039659 |
| 12034 | 0.009390 | 0.018673 |
| 12019 | 0.014193 | 0.039296 |
| 12000 | 0.014050 | 0.040630 |
| 11996 | 0.017409 | 0.051823 |
| 11976 | 0.015490 | 0.021507 |
| 11973 | 0.017012 | 0.022869 |
| 11969 | 0.014698 | 0.004180 |
| 11919 | 0.016897 | 0.011205 |
| 11915 | 0.015187 | 0.004898 |
| 11892 | 0.008932 | 0.001296 |
| 11876 | 0.014408 | 0.009473 |
| 11872 | 0.018896 | 0.008447 |
| 11822 | 0.023312 | 0.009941 |
| 11818 | 0.029877 | 0.021024 |
| 11814 | 0.031454 | 0.017436 |
| 11811 | 0.013878 | 0.003388 |
| 11799 | 0.012218 | 0.000976 |
| 11795 | 0.029877 | 0.021024 |
| 11791 | 0.027819 | 0.018584 |
| 11787 | 0.028750 | 0.019623 |
| 11772 | 0.011377 | 0.007962 |
| 11768 | 0.020593 | 0.017708 |
| 11764 | 0.018695 | 0.014589 |
| 11691 | 0.012978 | 0.009019 |
| 11687 | 0.011224 | 0.007745 |
| 11525 | 0.010380 | 0.012518 |
| 11502 | 0.017490 | 0.025658 |
| 11498 | 0.026037 | 0.017685 |
| 11494 | 0.015444 | 0.020679 |
| 11317 | 0.003346 | 0.056311 |
| 11313 | 0.001520 | 0.058640 |

This range separates the three groups perfectly, but operates in a zone of the spectrum that is very subject to variations deriving simply from the color of the samples.

Another analysis was carried out in the part of the spectrum between 4050 and 7150 cm⁻¹: the graphical results of the projection of the cases on the first two PCA components are shown in fig. 6.

Hereafter we show the weight of the variables on the PCA factors for this analysis.

| | Factor 1 | Factor 2 | Factor 3 |
|---|---|---|---|
| 7186 | 0.009395 | 0.013425 | 0.002298 |
| 7182 | 0.006612 | 0.019082 | 0.023341 |
| 7178 | 0.006051 | 0.019316 | 0.019963 |
| 7174 | 0.007022 | 0.025083 | 0.012149 |
| 7159 | 0.007517 | 0.015081 | 0.009159 |
| 6075 | 0.008780 | 0.007449 | 0.028322 |
| 6071 | 0.009821 | 0.006658 | 0.022457 |
| 6067 | 0.007084 | 0.015377 | 0.003024 |
| 6063 | 0.004687 | 0.008272 | 0.009477 |
| 6056 | 0.004575 | 0.002739 | 0.036257 |
| 5878 | 0.010197 | 0.000636 | 0.000940 |
| 5863 | 0.011363 | 0.002200 | 0.014462 |
| 5793 | 0.006832 | 0.024728 | 0.012575 |
| 5790 | 0.013900 | 0.003547 | 0.000691 |
| 5786 | 0.013151 | 0.000086 | 0.000866 |
| 5782 | 0.009051 | 0.020748 | 0.002868 |
| 5778 | 0.008412 | 0.022561 | 0.000655 |
| 5774 | 0.008477 | 0.027632 | 0.005813 |
| 5770 | 0.004817 | 0.033011 | 0.035264 |
| 5766 | 0.007210 | 0.029295 | 0.015941 |
| 5763 | 0.005296 | 0.022229 | 0.007807 |
| 5751 | 0.011516 | 0.000121 | 0.003263 |
| 5747 | 0.007004 | 0.004039 | 0.000269 |
| 5743 | 0.012215 | 0.000003 | 0.012690 |
| 5739 | 0.010682 | 0.007898 | 0.003340 |
| 5736 | 0.005878 | 0.000010 | 0.014055 |
| 5728 | 0.009179 | 0.001035 | 0.017141 |
| 5682 | 0.013086 | 0.004179 | 0.008912 |
| 5670 | 0.007022 | 0.025083 | 0.012149 |
| 5651 | 0.005161 | 0.034079 | 0.014393 |
| 5643 | 0.009280 | 0.012429 | 0.004413 |
| 5639 | 0.009096 | 0.003802 | 0.006849 |
| 5635 | 0.006959 | 0.022727 | 0.000753 |
| 5601 | 0.004719 | 0.003672 | 0.027524 |
| 5597 | 0.005552 | 0.011953 | 0.000169 |
| 5026 | 0.007529 | 0.001181 | 0.000078 |
| 5018 | 0.005211 | 0.002310 | 0.037125 |
| 4694 | 0.005656 | 0.013399 | 0.004605 |
| 4671 | 0.007702 | 0.004908 | 0.000559 |
| 4559 | 0.005396 | 0.000373 | 0.021712 |
| 4555 | 0.009471 | 0.011727 | 0.013919 |
| 4551 | 0.011028 | 0.008958 | 0.005527 |
| 4548 | 0.006526 | 0.011514 | 0.032647 |
| 4521 | 0.007653 | 0.006362 | 0.032219 |
| 4517 | 0.007341 | 0.011928 | 0.029392 |
| 4505 | 0.009465 | 0.002369 | 0.020685 |
| 4501 | 0.006472 | 0.006548 | 0.059723 |
| 4494 | 0.005665 | 0.000538 | 0.031961 |
| 4490 | 0.010198 | 0.009827 | 0.000214 |
| 4486 | 0.012898 | 0.008748 | 0.002682 |
| 4482 | 0.012405 | 0.001790 | 0.000004 |
| 4478 | 0.008801 | 0.004254 | 0.020702 |
| 4467 | 0.009767 | 0.001645 | 0.024035 |
| 4459 | 0.005069 | 0.000118 | 0.003306 |
| 4428 | 0.001651 | 0.023254 | 0.003003 |
| 4424 | 0.007847 | 0.011091 | 0.024211 |
| 4420 | 0.005907 | 0.005790 | 0.050691 |
| 4416 | 0.005907 | 0.005790 | 0.050691 |
| 4413 | 0.012369 | 0.002502 | 0.009556 |
| 4405 | 0.009897 | 0.004520 | 0.002445 |
| 4382 | 0.012776 | 0.008040 | 0.008103 |
| 4378 | 0.013105 | 0.005239 | 0.016604 |
| 4374 | 0.013471 | 0.000393 | 0.004318 |
| 4370 | 0.013455 | 0.000339 | 0.002377 |
| 4366 | 0.012244 | 0.002117 | 0.002632 |
| 4362 | 0.009742 | 0.013060 | 0.006773 |
| 4351 | 0.006389 | 0.032911 | 0.000241 |
| 4347 | 0.009626 | 0.029073 | 0.000546 |
| 4343 | 0.014028 | 0.011979 | 0.000827 |
| 4339 | 0.016554 | 0.004732 | 0.001398 |
| 4335 | 0.017709 | 0.000015 | 0.001997 |
| 4332 | 0.016365 | 0.006873 | 0.000008 |
| 4328 | 0.013237 | 0.020433 | 0.000164 |
| 4324 | 0.009113 | 0.038910 | 0.000141 |
| 4312 | 0.011280 | 0.023818 | 0.003911 |
| 4308 | 0.016632 | 0.002338 | 0.000179 |
| 4305 | 0.017228 | 0.001126 | 0.000286 |
| 4301 | 0.016159 | 0.006825 | 0.000009 |
| 4297 | 0.016374 | 0.006845 | 0.000400 |
| 4293 | 0.016416 | 0.004359 | 0.000229 |
| 4289 | 0.016550 | 0.002437 | 0.000051 |
| 4285 | 0.015695 | 0.001960 | 0.000388 |
| 4281 | 0.013028 | 0.010154 | 0.000599 |
| 4278 | 0.006813 | 0.035145 | 0.001100 |
| 4270 | 0.010739 | 0.025137 | 0.003281 |
| 4266 | 0.015209 | 0.009010 | 0.000853 |
| 4262 | 0.017156 | 0.000163 | 0.000824 |
| 4258 | 0.017536 | 0.000189 | 0.000250 |
| 4254 | 0.015394 | 0.008400 | 0.000019 |
| 4251 | 0.013025 | 0.015607 | 0.000110 |
| 4239 | 0.014566 | 0.006721 | 0.000256 |
| 4235 | 0.016025 | 0.000123 | 0.000012 |
| 4231 | 0.014052 | 0.005848 | 0.005332 |
| 4227 | 0.010734 | 0.016828 | 0.001477 |
| 4200 | 0.007172 | 0.001252 | 0.017399 |
| 4096 | 0.006282 | 0.010370 | 0.012303 |
| 4081 | 0.007775 | 0.000003 | 0.008312 |
| 4077 | 0.008369 | 0.000034 | 0.006878 |
| 4073 | 0.010604 | 0.000002 | 0.013108 |
| 4069 | 0.005950 | 0.019559 | 0.004360 |

## Claims

1. Method to evaluate the quality of cork, comprising:
- supplying a plurality of calibration samples made of cork, both not contaminated and contaminated by a fungal metabolism;
- dividing the calibration samples into a plurality of quality categories as a function of the attack by the fungal metabolism;
- spectroscopic sampling analysis in the near-infrared of said calibration samples to identify, from the processing of the respective characteristic spectrum, identifying reference parameters of each of said quality categories and correlated to the attack by the fungal metabolism;
- spectroscopic analysis in the near-infrared band of at least one product (11) made of cork, which must be evaluated, to detect a characteristic spectrum of said cork of said product (11);
- processing said characteristic spectrum of said product (11) made of cork to evaluate at least one conditioning parameter of the quality of said cork correlated to the attack by the fungal metabolism;
- comparing said conditioning parameter with said reference parameters, to identify whether said product (11) belongs to one of said quality categories;
- selection of said product (11) as a function of said comparison, to associate it to one of said quality categories.

2. Method as in any claim hereinbefore, wherein dividing of the calibration samples is carried out by means of a technique of sensory and/or chromatographic analysis.

3. Method as in any claim hereinbefore, wherein the evaluation of said conditioning parameter of the quality of the cork provides to identify at least one indicator of microstructural/physical and/or chemical modification of said cork.

4. Method as in claim 3, wherein said at least one indicator of microstructural/physical modification concerns the development of at least one of anisole, geosmin, isoborneol, guaiacol, or octenone.

5. Method as in claim 3 or 4, wherein said at least one indicator of microstructural/physical modification concerns the development of 2, 4, 6-trichloroanisole in the cork.

6. Method as in any claim hereinbefore, wherein processing said characteristic spectrum of the cork provides to implement techniques for the statistical processing of the data detected in multivariate mode in order to determine said reference parameters and conditioning parameters of the quality of the cork.

7. Method as in claim 6, wherein said multivariate statistical processing techniques comprise at least one of either the "Principal Component Analysis", "Partial Least Squares" and "Soft Independent Modelling of Class Analogies (SIMCA)" techniques.

8. Method as in any claim hereinbefore, wherein during said processing, it provides to analyze a wavelength range of said characteristic spectrum comprised between 4500 cm⁻¹ and 10000 cm⁻¹.

9. Method as in any claim hereinbefore, wherein said spectrums, both of the calibration samples and of the products (11), are subjected to mathematical filters.

10. Apparatus to evaluate the quality of cork comprising a spectroscopic analysis device (15) configured to detect characteristic spectrums of calibration samples made of cork, both not contaminated and contaminated by a fungal metabolism, and at least a processing device (18) configured to process said characteristic spectrums, wherein said spectroscopic analysis device (15) is configured to operate in the near-infrared to detect said characteristic spectrums of calibration samples made of cork, and at least a characteristic spectrum of at least one product (11) made of cork that is to be evaluated, wherein said apparatus comprises a database containing information concerning quality categories, divided as a function of the attack by the fungal metabolism, into which said calibration samples are divided, wherein said processing device (18) is configured to identify, from the characteristic spectrum of each calibration sample, reference parameters of each of said quality categories correlated to the attack by the fungal metabolism and, from the characteristic spectrum of each product (11) that is to be evaluated, to evaluate at least one conditioning parameter of the quality of said cork correlated to the attack by the fungal metabolism, said processing device (18) being configured to compare said conditioning parameter with said reference parameters, and wherein said apparatus comprises a selector device (19) configured to select said product (11) as a function of said comparison, and to associate it to one of said quality categories contained in said database.

## Patentansprüche

1. Verfahren, um die Qualität von Kork zu evaluieren, welches aufweist:
- Bereitstellen einer Mehrzahl sowohl von nicht kontaminierten als auch mittels eines Pilzstoffwechsels kontaminierten Kalibrierungsproben, welche aus Kork hergestellt sind,
- Einteilen der Kalibrierungsproben in eine Mehrzahl von Qualitätskategorien als eine Funktion des Angriffs mittels des Pilzstoffwechsels,
- spektroskopische Probenanalyse im nahen Infrarot der besagten Kalibrierungsproben, um aus der Verarbeitung des jeweiligen charakteristischen Spektrums identifizierende Referenzparameter jeder der besagten Qualitätskategorien zu identifizieren, welche mit dem Angriff mittels des Pilzstoffwechsels korreliert sind,
- spektroskopische Analyse im Nahes-Infrarot-Band von zumindest einem aus Kork hergestellten Produkt (11), welches evaluiert werden muss, um ein charakteristisches Spektrum des besagten Korks des besagten Produkts (11) zu detektieren,
- Verarbeiten des besagten charakteristischen Spektrums des besagten aus Kork hergestellten Produkts (11), um zumindest einen Konditionierungsparameter der Qualität des besagten Korks zu evaluieren, welcher mit dem Angriff mittels des Pilzmetabolismus korreliert ist,
- Vergleichen des besagten Konditionierungsparameters mit den besagten Referenzparametern, um zu identifizieren, ob das besagte Produkt (11) zu einer der besagten Qualitätskategorien gehört,
- Auswählen des besagten Produkts (11) als eine Funktion des besagten Vergleichs, um es mit einer der besagten Qualitätskategorien zu assoziieren.

2. Verfahren gemäß irgendeinem vorherigen Anspruch, wobei das besagte Einteilen der Kalibrierungsproben mittels einer sensorischen Technik und/oder chromatographischer Analyse durchgeführt wird.

3. Verfahren gemäß irgendeinem vorherigen Anspruch, wobei das Evaluieren des besagten Konditionierungsparameters der Qualität des Korks bereitstellt, zumindest einen Indikator für eine mikrostrukturelle/physikalische und/oder chemische Modifikation des besagten Korks zu identifizieren.

4. Verfahren gemäß Anspruch 3, wobei der besagte zumindest eine Indikator für eine mikrostrukturelle/physikalische Modifikation die Entwicklung von zumindest einem von Anisol, Geosmin, Isoborneol, Guajakol oder Octenon betrifft.

5. Verfahren gemäß Anspruch 3 oder 4, wobei der besagte zumindest eine Indikator für eine mikrostrukturelle/physikalische Modifikation die Entwicklung von 2, 4, 6-Trichloranisol im Kork betrifft.

6. Verfahren gemäß irgendeinem vorherigen Anspruch, wobei das Verarbeiten des besagten charakteristischen Spektrums des Korks bereitstellt, Techniken zum statistischen Verarbeiten der in einem multivariaten Modus detektierten Daten zu implementieren, um die besagten Referenzparameter und Konditionierungsparameter der Qualität des Korks zu ermitteln.

7. Verfahren gemäß Anspruch 6, wobei die besagten multivariaten statistischen Verarbeitungstechniken zumindest eine von der "Hauptkomponentenanalyse"-, der "Partielle kleinste Quadrate"- und der "Weiches unabhängiges Modellieren von Klassenanalogien (SIMCA)"-Technik aufweisen.

8. Verfahren gemäß irgendeinem vorherigen Anspruch, wobei es während des besagten Verarbeitens bereitstellt, einen Wellenlängenbereich des besagten charakteristischen Spektrums zu analysieren, welcher zwischen 4500 cm⁻¹ und 10000 cm⁻¹ liegt.

9. Verfahren gemäß irgendeinem vorherigen Anspruch, wobei die besagten Spektren sowohl der Kalibrierungsproben als auch der Produkte (11) mathematischen Filtern unterzogen werden.

10. Vorrichtung, um die Qualität von Kork zu evaluieren, welche aufweist eine spektroskopische Analysevorrichtung (15), welche eingerichtet ist, um charakteristische Spektren sowohl von nicht kontaminierten als auch mittels eines Pilzstoffwechsels kontaminierten Kalibrierungsproben, welche aus Kork hergestellt sind, zu detektieren, und zumindest eine Verarbeitungsvorrichtung (18), welche eingerichtet ist, um die besagten charakteristischen Spektren zu verarbeiten, wobei die besagte spektroskopische Analysevorrichtung (15) eingerichtet ist, um im nahen Infrarot zu arbeiten, um die besagten charakteristischen Spektren von aus Kork hergestellten Kalibrierungsproben und zumindest ein charakteristisches Spektrum von zumindest einem aus Kork hergestellten Produkt (11), welches zu evaluieren ist, zu detektieren, wobei die besagte Vorrichtung eine Datenbank aufweist, welche Informationen enthält, welche als eine Funktion des Angriffs mittels des Pilzstoffwechsels eingeteilte Qualitätskategorien betreffen, in welche die besagten Kalibrierungsproben eingeteilt sind, wobei die besagte Verarbeitungsvorrichtung (18) eingerichtet ist, um aus dem charakteristischen Spektrum jeder Kalibrierungsprobe Referenzparameter jeder der besagten Qualitätskategorien zu identifizieren, welche mit dem Angriff mittels des Pilzstoffwechsels korreliert sind, und um aus dem charakteristischen Spektrum jedes Produkts (11), welches zu evaluieren ist, zumindest einen Konditionierungsparameter der Qualität des besagten Korks zu evaluieren, welcher mit dem Angriff mittels des Pilzstoffwechsels korreliert ist, wobei die besagte Verarbeitungsvorrichtung (18) eingerichtet ist, um den besagten Konditionierungsparameter mit den besagten Referenzparameters zu vergleichen, und wobei die besagte Vorrichtung eine Auswahlvorrichtung (19) aufweist, welche eingerichtet ist, um das besagte Produkt (11) als eine Funktion des besagten Vergleichs auszuwählen, und um es mit einer der besagten Qualitätskategorien zu assoziieren, welche in der besagten Datenbank enthalten sind.

## Revendications

1. Procédé d'évaluation de la qualité de liège, comprenant les étapes consistant à :
fournir une pluralité d'échantillons d'étalonnage réalisés en liège, à la fois non contaminés et contaminés par un métabolisme fongique ;
diviser des échantillons d'étalonnage en une pluralité de catégories de qualité en fonction de l'attaque par le métabolisme fongique ;
analyser l'échantillonnage de manière spectroscopique dans le proche infrarouge desdits échantillons d'étalonnage pour identifier, à partir du traitement du spectre caractéristique respectif, des paramètres de référence d'identification de chacune desdites catégories de qualité et corrélés à l'attaque par le métabolisme fongique ;
analyser de manière spectroscopique dans la bande du proche infrarouge au moins un produit (11) réalisé en liège, qui doit être évalué, pour détecter un spectre caractéristique dudit liège dudit produit (11) ;
traiter ledit spectre caractéristique dudit produit (11) réalisé en liège pour évaluer au moins un paramètre de conditionnement de la qualité dudit liège corrélé à l'attaque par le métabolisme fongique ;
comparer ledit paramètre de conditionnement avec lesdits paramètres de référence, pour identifier si ledit produit (11) appartient à l'une desdites catégories de qualité ;
sélectionner ledit produit (11) en fonction de ladite comparaison, pour l'associer à l'une desdites catégories de qualité.

2. Procédé selon l'une quelconque des revendications précédentes, dans lequel ladite division des échantillons d'étalonnage est effectuée au moyen d'une technique d'analyse sensorielle et/ou chromatographique.

3. Procédé selon l'une quelconque des revendications précédentes, dans lequel l'évaluation dudit paramètre de conditionnement de la qualité du liège permet d'identifier au moins un indicateur de modification micro structurelle/physique et/ou chimique dudit liège.

4. Procédé selon la revendication 3, dans lequel ledit au moins un indicateur de modification microstructurelle/physique concerne le développement d'au moins un parmi l'anisole, la géosmine, l'isobornéol, le gaïacol ou l'octénone.

5. Procédé selon la revendication 3 ou 4, dans lequel ledit au moins un indicateur de modification microstructurelle/physique concerne le développement de 2,4,6-trichloroanisole dans le liège.

6. Procédé selon l'une quelconque des revendications précédentes, dans lequel le traitement dudit spectre caractéristique du liège permet de mettre en œuvre des techniques pour le traitement statistique des données détectées en mode multivarié afin de déterminer lesdits paramètres de référence et lesdits paramètres de conditionnement de la qualité du liège.

7. Procédé selon la revendication 6, dans lequel lesdites techniques de traitement statistique multivarié comprennent au moins une parmi « Analyse de Composant Principal », « Moindres Carrés Partiels » et « Modélisation Indépendante Douce d'Analogies de Classe (SIMCA) ».

8. Procédé selon l'une quelconque des revendications précédentes, dans lequel, pendant ledit traitement, il prévoit d'analyser une plage de longueur d'onde dudit spectre caractéristique comprise entre 4 500 cm⁻¹ et 10 000 cm⁻¹.

9. Procédé selon l'une quelconque des revendications précédentes, dans lequel lesdits spectres, à la fois des échantillons d'étalonnage et des produits (11), sont soumis à des filtres mathématiques.

10. Appareil pour évaluer la qualité de liège comprenant un dispositif d'analyse spectroscopique (15) configuré pour détecter des spectres caractéristiques d'échantillons d'étalonnage réalisés en liège, à la fois non contaminés et contaminés par un métabolisme fongique, et au moins un dispositif de traitement (18) configuré pour traiter lesdits spectres caractéristiques, dans lequel ledit dispositif d'analyse spectroscopique (15) est configuré pour fonctionner dans le proche infrarouge afin de détecter lesdits spectres caractéristiques d'échantillons d'étalonnage réalisés en liège et au moins un spectre caractéristique d'au moins un produit (11) réalisé en liège qui doit être évalué, dans lequel ledit appareil comprend une base de données contenant des informations concernant des catégories de qualité, divisées en fonction de l'attaque par le métabolisme fongique, dans lesquelles lesdits échantillons d'étalonnage sont divisés, dans lequel ledit dispositif de traitement (18) est configuré pour identifier, à partir du spectre caractéristique de chaque échantillon d'étalonnage, des paramètres de référence de chacune desdites catégories de qualité corrélés à l'attaque par le métabolisme fongique et, à partir du spectre caractéristique de chaque produit (11) qui doit être évalué, pour évaluer au moins un paramètre de conditionnement de la qualité dudit liège corrélé à l'attaque par le métabolisme fongique, ledit dispositif de traitement (18) étant configuré pour comparer ledit paramètre de conditionnement auxdits paramètres de référence, et dans lequel ledit appareil comprend un dispositif de sélection (19) configuré pour sélectionner ledit produit (11) en fonction de ladite comparaison, et pour l'associer à l'une desdites catégories de qualité contenues dans ladite base de données.
